Europäisches Patentamt

European Patent Office

Office européen des brevets

(11) Numéro de publication: **0 051 709**
**B1**

(12) **FASCICULE DE BREVET EUROPEEN**

(45) Date de publication du fascicule du brevet: **20.03.85**

(21) Numéro de dépôt: **80401598.0**

(22) Date de dépôt: **07.11.80**

(51) Int. Cl.⁴: **A 61 F 13/20, A 61 M 31/00**

(54) Tampon contraceptif, ensemble d'un tampon et d'un dispositif applicateur et procédé de préparation du tampon.

(43) Date de publication de la demande:
**19.05.82 Bulletin 82/20**

(45) Mention de la délivrance du brevet:
**20.03.85 Bulletin 85/12**

(84) Etats contractants désignés:
**AT BE CH DE GB IT LI LU NL SE**

(56) Documents cités:
**FR-A-2 070 061**
**US-A-2 687 729**
**US-A-3 762 414**
**US-A-3 916 898**
**US-A-3 918 452**

(73) Titulaire: **ATLANTIC PHARMACEUTICAL PRODUCTS**
**Ballymakealy 12 Lucan**
**Dublin (IE)**

(72) Inventeur: **Langlois, Pierre**
**24, rue de Clichy**
**F-75009 Paris (FR)**
Inventeur: **Mollet, Marc**
**38, rue des Chevaliers de St Jean**
**F-91100 Corbeil Essonnes (FR)**

(74) Mandataire: **Chenard, René Marcel et al**
**Cabinet BUGNION ASSOCIES 116, boulevard Haussmann**
**F-75008 Paris (FR)**

Courier Press, Leamington Spa, England.

EP 0 051 709 B1

# Description

La présente invention concerne un nouveau moyen contraceptif et plus particulièrement un tampon contraceptif à action spermicide.

Outre les moyens contraceptifs mécaniques, de type intrautérin ou préservatif féminin, on connaît différents types de contraceptifs à action ponctuelle utilisant des compositions spermicides se présentant sous la forme de crème ou de gel en tube ou des ovules ou capsules de gel spermicides. Sous les formes ainsi proposées, l'application du gel ou de la crème s'avère parfois délicate, voire non satisfaisante et, dans tous les cas, la composition spermicide ainsi appliquée présente une fusion ou une dilution trop rapide nuisant grandement à son efficacité. D'autre part, avec ces présentations, l'application de la composition spermicide, qui s'effectue au moyen d'un doigt peut présenter des risques de contamination, la présentation sous forme d'ovules ou de capsules posant en outre des problèmes de conditionnement dans des alvéoles hermétiques.

On connaît également, notamment du brevet US—A—3 918 452, un moyen contraceptif sous forme de tampon, à base de composition spermicide en imprégnation sur un élément d'éponge synthétique en matériau cellulaire, ce qui permet une mise en place simple et assurant une libération progressive de la composition spermicide, en combinant tout à la fois les effets d'un contraceptif mécanique et d'un contraceptif à action spermicide.

On constate cependant, avec ce type de tampon contraceptif, que la durée de l'action spermicide n'est pas toujours satisfaisante.

L'invention vise à palier cet inconvénient en proposant un tampon contraceptif à double action mécanique et spermicide, comportant un élément d'éponge synthétique en matériau cellulaire imprégné d'une composition spermicide, caractérisé en ce que le matériau de l'éponge comprend des cellules "semi-ouvertes", c'est-à-dire des cellules en partie ouvertes qui sont reliées les unes aux autres sans cependan former un système cohérent de canaux, et en ce que la composition spermicide est mélangée à un milieu retard. Grâce à ces caractéristiques, l'invention permet d'obtenir une durée satisfaisante pour l'action spermicide du tampon contraceptif.

Par ailleurs, l'invention concerne aussi un procédé de préparation d'un tel tampon contraceptif, dans lequel on met en forme une éponge, on stérilise celle-ci, on prépare une composition contraceptive et on imprègne l'éponge stérilisée de cette composition, comme il est connu du brevet US—A—3916898. Le procédé selon l'invention est caractérisé par le fait qu'on utilise une éponge comprenant des cellules en partie ouvertes et en ce que la préparation de la composition contraceptives consiste à mélanger une composition spermicide avec un milieu retard stérilisé.

Selon une autre caractéristique de la présente invention, l'éponge peut éventuellement être maintenue à l'état comprimé et le mélange dont elle est imprégnée être desséché par un procédé adéquant, par exemple lyophilisé.

La présente invention concerne un contraceptif à action ponctuelle et se distingue tant par la conformation et la structure de l'éponge, par les substances dont l'éponge est imprégnée, que par le mode d'utilisation pour lequel cette forme et cette composition sont tout spécialement combinées en vue d'aboutir à un produit spécifique répondant aux exigences de sécurité requises et adapté à l'environnement pour lequel il est destiné pour l'obtention d'un effet contraceptif particulièrement efficace. En effet, la combinaison d'une éponge conformée, éventuellement comprisée et expansible sous l'effet de l'humidité pour s'adapter à la géométrie du vagin, constituée d'un matériau synthétique à cellules semi-ouvertes afin de ne libérer que progressivement les produits dont elle est imprégnée, et de la composition de ces produits réalise un moyen contraceptif facilement stockable, de mise en place aisée, réalisant, sous l'effet des sécrétions internes de la femme ou d'une humectation préalable ou d'un état d'humidité préexistant, tout à la fois un gonflement, permettant d'occulter le col utérin et une libération progressive et échelonnée dans le temps, à action immédiate et continue, de la composition spermicide, soumettant ainsi les spermatozoïdes éjaculés à un barrage mécanique et à une action biochimique garantissant l'effet recherché.

Selon une caractéristique de la présente invention, le tampon comporte un moyen d'extraction réalisé en un cordon tissé anti-mèche, se présentant avantageusement sous la forme d'une boucle fermée dont le noeud d'assemblage est noyé dans la masse de l'éponge, permettant ainsi, sans nuire aux qualités de diffusion progressive de la composition spermicide, d'extraire très facilement le tampon au moment opportun et lui permettre notamment de pratiquer une toilette intime ultérieure.

Le tampon selon la présente invention peut en outre être avantageusement fourni avec un dispositif applicateur, permettant ainsi une mise en place hygiénique aisée et efficace, à la façon des tampons cataméniaux.

D'autres caractéristiques et avantages de la présente invention ressortiront de la description suivante d'un mode de réalisation, donné à titre illustratif mais nullement limitatif, faite en relation avec les dessins annexés sur lesquels:

la figure 1 est une vue schématique en perspective d'un tampon contraceptif selon un mode particulier de réalisation de la présente invention, et

la figure 2 est une vue en perspective d'un tel tampon logé dans un dispositif applicateur conçu à cet effet.

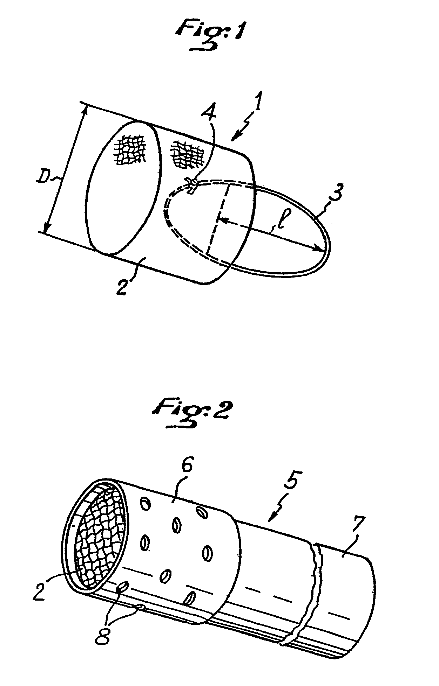

Comme représenté à la figure 1, le contraceptif selon la présente invention se présente sous la forme d'un tampon, généralement référencé 1, constitué d'un corps 2, en un matériau cellulaire comprenant des cellules semi-ouvertes et, avantageusement, uniquement des cellules semi-ouvertes, imprégné d'un mélange d'une composition spermicide, d'un milieu retard et, éventuellement, d'adjuvants ou d'additifs autorisés en pharmacologie et comprenant par exemple des agents antiseptiques. Le corps 2, dénommé éponge dans la présente description, est réalisé en un matériau présentant une parfaite tolérance vis-à-vis des muqueuses vaginales et comprenant des pores de dimensions comprises entre environ 5/10$^e$ mm et 1 mm. Dans sa présentation normale, le tampon 1 peut se présenter sous une forme comprimée, facilitant ainsi son introduction, son humidification ou une humidification complémentaire, puis son imprégnation par les secrétions vaginales concourant éventuellement à son expansion après sa mise en place dans le vagin.

On confère à l'éponge 2 une forme anatomique lui permettant de se loger aisément au voisinage du fond du vagin près du col de l'utérus sans se placer dans une mauvaise position et en gênant le moins possible la femme qu'il occupe ou le sexe du partenaire éventuel lors de l'accouplement. De préférence, l'éponge 2 aura une forme sphérique ou pseudo-sphérique avec une partie centrale cylindrique mais toutefois, comme représenté sur la figure 1, cette éponge peut présenter, pour des commodités de réalisation, la forme d'un cylindre droit, dont la hauteur est voisine du diamètre D dans la configuration expansée du tampon, ce diamètre D étant compris entre environ 4 cm et 4,5 cm.

Comme déjà mentionné ci-dessus, pour permettre une extraction aisée de l'éponge, le tampon comprend un organe d'extraction 3, réalisé en une tresse de coton ou d'un polyamide, ces fibres étant tissées anti-mèche, de façon connue en soi, pour éviter l'effet de mèche. Conformément à un aspect préférentiel de la présente invention, le cordon tissé 3 se présente sous la forme d'une boucle fermée, le noeud d'assemblage 4 étant noyé dans la masse de l'éponge 2. Selon une caractéristique importante de la présente invention, la flèche maximale / de la portion extérieure libre de la boucle est comprise entre environ 3 et environ 5 cm.

Le matériau cellulaire constitutif de l'éponge 2 peut être à base de fibrine, ou en polyéthylène expansé de poids moléculaire bas ou moyen, compris par exemple entre 15.000 et 35.000, et totalement débarrassé des résidus d'agent moussant. Dans un mode de réalisation préférentiel, l'éponge 2 est réalisée en alcool polyvinylique insolubilisé au formol puis rincé. Ce dernier matériau présente notamment l'avantage de garder, après compression, la forme comprimée en l'absence d'humidité et de se regonfler rapidement, une fois humidifié.

La composition spermicide dont est imprégnée l'éponge 2 comprend avantageusement de l'alkylbenzalkonium, d'autres substances spermicides, telles que par exemple le chlorure de benzéthonium et le nitrate de phénylmercure, pouvant également être utilisées. Le milieu retard, permettant d'étaler dans le temps la mobilisation des principes actifs de la composition spermicide et de prolonger leur action, est constitué par exemple par un gel stérile de carboxyméthylcellulose, stabilisé par un conservateur tel que le para-hydroxybenzoate de méthyle.

La préparation du tampon contraceptif selon la présente invention s'effectue comme suit. L'éponge 2 est mise en forme, par exemple par moulage du matériau cellulaire, puis stérilisée, par exemple dans un autoclave, avec un gaz stérilisant ou un rayonnement adéquat, à la suite de quoi l'éponge ainsi stérilisée est imprégnée du mélange susmentionné en la plongeant dans une solution de ce mélange, puis en l'essorant éventuellement modérément. Selon une variante, l'éponge imprégnée est ensuite soumise à dessication, par exemple par lyophilisation avec congélation et sublimation, puis comprimée au format requis pour permettre son introduction aisée dans le vagin.

Le tampon ainsi réalisé est imputrescible aux liquides organiques de sécrétions, est inaltérable à la chaleur et au froid, notamment lorsqu'il est en état de dessication; il est non résorbable, neutre et compatible avec les tissus de la muqueuse vaginale. Il ne présente notamment vis-à-vis de celle-ci aucun effet d'adhérence et sa souplesse, en configuration expansée, le rend pratiquement imperceptible par l'utilisatrice et son partenaire.

Lorsque le tampon est sous une forme sèche et comprimée avant usage, il permet l'utilisation d'un dispositif applicateur, et, notamment du dispositif applicateur particulier représenté sur la figure 2. Le dispositif applicateur 5, de conception classique, constitué d'un tube cylindrique 6 destiné à recevoir le tampon comprimé 2 et d'un tube poussoir 7, comprend toutefois, à la différence des dispositifs applicateurs existants, des orifices ou évidements 8 formés dans la paroi du tube cylindrique 6. Il est ainsi possible, avec ce dispositif applicateur, de tremper préalablement le tube cylindrique 6 contenant le tampon sec et comprimé 2 dans de l'eau pour réaliser une pré-expansion du matérieu cellulaire de l'éponge 2, la mise en place du tampon s'effectuant de façon traditionnelle, son gonflement ainsi amorcé le randant quasiment immédiatement utilisable, indépendamment de la quantité des sécrétions internes de la femme. Un tel tampon peut ainsi être utilisé moins d'une minute après son introduction, ses caractéristiques contraceptives se prolongeant pendant un délai supérieur à 5 heures.

## Revendications

1. Tampon contraceptif à double action mécanique et spermicide comportant un élément d'éponge synthétique (2) en matériau cellulaire imprégné d'une composition spermicide caractérisé en ce que le matériau de l'éponge comprend des cellules en partie ouvertes qui sont reliées les unes aux autres sans cependant former un système cohérent de canaux et en ce que la composition spermicide est mélangée à un milieu retard.

2. Tampon selon la revendication 1, caractérisé en ce que l'éponge (2) est maintenue dans un état sec et comprimé, le mélange étant au préalable desséché.

3. Tampon selon la revendication 1 ou la revendication 2, caractérisé en ce qu'il comporte un organe d'extraction (3) relié à l'éponge et réalisé en un cordon tissé anti-mèche.

4. Tampon selon la revendication 3, caractérisé en ce que le cordon se présente sous la forme d'une boucle fermée, le noeud d'assemblage (4) étant noyé dans la masse de l'éponge (2).

5. Tampon selon la revendication 4, caractérisé en ce que la flèche maximale de la portion de boucle libre extérieure est comprise entre environ 3 et 5 cm.

6. Tampon selon l'une quelconque des revendications 1 à 5, caractérisé en ce que l'éponge (2) est conformée en forme de cylindre, le diamètre de l'éponge à l'état expansé étant de l'ordre de 4 à 4,5 cm. et sa hauteur, du même ordre de grandeur.

7. Tampon selon l'une quelconque des revendications 1 à 6, caractérisé en ce que l'éponge (2) est réalisée en polyéthylène expansé, de poids moléculaire compris entre environ 15.000 et 35.000 et exempt de résidue d'agent moussant.

8. Tampon selon l'une quelconque des revendications 1 à 6, caractérisé en ce que l'éponge (2) est réalisée en alcool polyvinylique insolubilisé.

9. Tampon selon l'une quelconque des revendications 1 à 6, caractérisé en ce que l'éponge (2) est réalisée en fibrine.

10. Tampon selon l'une quelconque des revendications 1 à 9, caractérisé en ce que la composition spermicide comprend de l'alkylbenzalkonium.

11. Tampon selon l'une quelconque des revendications 1 à 10, caractérisé en ce que le milieu retard comprend un gel stérile de carboxyméthylcellulose.

12. Ensemble d'un tampon selon l'une quelconque des revendications 1 à 11, et d'un dispositif applicateur télescopique, caractérisé en ce que le tampon (1) est logé dans un tube cylindrique (6) du dispositif applicateur (5).

13. Ensemble selon la revendication 12, caractérisé en ce que la paroi du tube cylindrique (6) est percée d'orifices (8).

14. Procédé de préparation d'un tampon selon l'une quelconque des revendications 1 à 11 dans lequel on met en forme une éponge (8), on stérilise celle-ci, on prépare une composition contraceptive et on imprègne l'éponge stérilisée de cette composition, caractérisé en ce qu'on utilise une éponge comprenant des cellules en partie ouverte et en ce que la préparation de la composition contraceptive consiste à mélanger une composition spermicide avec un milieu retard stérilisé.

15. Procédé selon la revendication 14, caractérisé en ce que, après l'étape d'imprégnation de l'éponge (2) celle-ci est desséchée et comprimée sous une forme convenable.

## Patentansprüche

1. Antikonzeptionstampon mit sowohl mechanischer als auch spermizider Wirkung, bestehend aus einem synthetischen schwammartigen Element (2) aus zellulärem Material, welches mit einer spermiziden Verbindung durchtränkt ist, dadurch gekennzeichnet, daß das Material des Schwammes teilweise offene Zellen enthält, die untereinander verbunden sind, ohne jedoch ein zusammenhängendes Kanalsystem zu bilden, und daß die spermizide Verbindung einem Verzögerungsmittel beigemischt ist.

2. Tampon nach Patentanspruch 1, dadurch gekennzeichnet, daß der Schwamm (2) im trockenen und komprimierten Zustand vorliegt, wobei die Mischung vorher getrocknet wurde.

3. Tampon nach Patentanspruch 1 oder 2, dadurch gekennzeichnet, daß er eine Ausziehvorrichtung (3) besitzt, die mit dem Schwamm verbunden ist und aus einem gegensträhnig gewebten Faden besteht.

4. Tampon nach Patentanspruch 3, dadurch gekennzeichnet, daß der Faden in Form einer geschlossenen Schlinge vorliegt, deren Verbindungsknoten (4) in der Masse des Schwammes (2) eingebettet ist.

5. Tampon nach Patentanspruch 4, dadurch gekennzeichnet, daß die maximale Abmessung des freien äußeren Teiles der Schlinge zwischen ungefähr 3 und 5 cm beträgt.

6. Tampon nach irgendeinem der Patentansprüche 1 bis 5, dadurch gekennzeichnet, daß der Schwamm (2) die Form eines Zylinders aufweist, wobei der Durchmesser des Schwammes im aufgequollenen Zustand in der Größenordnung von 4 bis 4,5 cm liegt, in der gleichen Größenordnung wie seine Höhe.

7. Tampon nach irgendeinem der Patentansprüche 1 bis 6, dadurch gekennzeichnet, daß der Schwamm (2) aus expandiertem Polyäthylen von eimen Molekulargewicht zwischen ungefähr 15.000 und 35.000 besteht und frei von Rückständen eines Schäumungsagens ist.

8. Tampon nach irgendeinem der Patentansprüche 1 bis 6, dadurch gekennzeichnet, daß der Schwamm (2) aus unlöslichem Polyvinylalkohol besteht.

9. Tampon nach irgendeinem der Patentansprüche 1 bis 6, dadurch gekennzeichnet, daß der Schwamm (2) aus einem Faserstoff besteht.

10. Tampon nach irgendeinem der Patentansprüche 1 bis 9, dadurch gekennzeichnet, daß die spermizide Zusammensetzung Alkylbenzalkonium enthält.

11. Tampon nach irgendeinem der Patentansprüche 1—10, dadurch gekennzeichnet, daß das Verzögerungsmittel ein steriles Carboxymethylcellulosegel enthält.

12. Tamponsatz nach irgendeinem der Patentansprüche 1 bis 11 mit einer zusammenschiebbaren Applikationsvorrichtung, dadurch gekennzeichnet, daß der Tampon (1) in einer zylindrischen Röhre (6) der Applikationsvorrichtung (5) eingelagert ist.

13. Tamponsatz nach Patentanspruch 12, dadurch gekennzeichnet, daß die Wand der zylindrischen Röhre (6) mit Öffnungen (8) durchsetzt ist.

14. Verfahren zur Herstellung eines Tampons nach irgendeinem der Patentansprüche 1 bis 11, bei welchem ein Schwamm (8) in die entsprechende Form gebracht wird, dieser sterilisiert, eine kontrazeptive Verbindung herstellt und der sterilisierte Schwamm mit dieser Verbindung durchtränkt wird, dadurch gekennzeichnet, daß ein Schwamm eingesetzt wird, der teilweise geöffnete Zellen enthält und daß die Herstellung der kontrazeptiven Verbindung darin besteht, daß eine spermizide Verbindung mit einem sterilisierten Verzögerungsmittel gemischt wird.

15. Verfahren nach Patentanspruch 14, dadurch gekennzeichnet, daß nach der Durchtränkung des Schwammes (2) dieser getrocknet und in einer geeigneten Form komprimiert wird.

## Claims

1. A contraceptive plug having a double mechanical and spermicide action comprising a synthetic sponge element (2) of a cellular material impregnated with a spermicide composition, characterised in that the sponge material comprises partly open cells which are connected to one another without however forming a coherent system of passageways and the spermicide composition is mixed with a delaying medium.

2. A plug according to claim 1, characterised in that the sponge (2) is maintained in a dry and compressed state, the mixture being previously dried.

3. A plug according to claim 1 or 2, characterised in that it comprises an extracting element (3) connected to the sponge and made from an anti-wick woven cord.

4. A plug according to claim 3, characterised in that the cord is in the form of a closed loop, the assembling knot (4) being embedded within the sponge (2).

5. A plug according to claim 4, characterised in that the maximum deflection of the free exterior loop portion is between about 3 and 5 cm.

6. A plug according to any one of the claims 1 to 5, characterised in that the sponge (2) is in the shape of a cylinder, the diameter of the sponge in the expanded state being on the order of 4 to 4.5 cm and its height being on the same order of magnitude.

7. A plug according to any one of the claims 1 to 6, characterised in that the sponge (2) is made from expanded polyethylene having a molecular weight between about 15,000 and 35,000 and devoid of foaming agent residues.

8. A plug according to any of the claims 1 to 6, characterised in that the sponge (2) is made from polyvinyl alcohol rendered insoluble.

9. A plug according to any one of the claims 1 to 6, characterised in that the sponge (2) is made from fibrin.

10. A plug according to any one of the claims 1 to 9, characterised in that the spermicide composition comprises alkylbenzalkonium.

11. A plug according to any one of the claims 1 to 10, characterised in that the delaying medium comprises a sterile gel of carboxymethylcellulose.

12. An assembly comprising a plug according to any one of the claims 1 to 11 and a telescopic applicator device, characterised in that the plug (1) is disposed within a cylindrical tube (6) of the applicator device (5).

13. An assembly according to claim 12, characterised in that the wall of the cylindrical tube (6) is provided with orifices (8).

14. A process for preparing a plug according to any one of the claims 1 to 11, comprising forming a sponge (8), sterilizing the latter, preparing a contraceptive composition and impregnating the sterilized sponge with this composition, characterised in that there is employed a sponge comprising partly open cells and the preparation of the contraceptive composition comprises mixing a spermicide composition with a sterilized delaying medium.

15. A process according to claim 14, characterised in that the sponge (2) is dried and compressed into a suitable shape after the sponge impregnating step.

*Fig:1*

*Fig:2*